(19) Europäisches Patentamt / European Patent Office / Office européen des brevets

(11) **EP 2 679 151 B1**

(12) **FASCICULE DE BREVET EUROPEEN**

(45) Date de publication et mention
de la délivrance du brevet:
**18.11.2020   Bulletin 2020/47**

(51) Int Cl.:
*A61B 5/04* *(2006.01)*      *G06F 3/01* *(2006.01)*
*A61B 5/0484* *(2006.01)*      *A61B 5/00* *(2006.01)*

(21) Numéro de dépôt: **13174104.3**

(22) Date de dépôt: **27.06.2013**

(54) **Procédé de localisation d'une activité cérébrale**

Verfahren zur Lokalisierung einer Gehirnaktivität

Method for locating brain activity

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:   **29.06.2012   FR 1256292**

(43) Date de publication de la demande:
**01.01.2014   Bulletin 2014/01**

(73) Titulaire: **COMMISSARIAT À L'ÉNERGIE
ATOMIQUE ET AUX
ÉNERGIES ALTERNATIVES
75015 Paris (FR)**

(72) Inventeurs:
• **Aksenova, Tetiana**
  **38120 Saint-Egrève (FR)**
• **Labyt, Etienne**
  **38950 St Martin le Vinoux (FR)**
• **Mishchenko, Ales**
  **192283 St Petersbourg (RU)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
• MATHIEU BOURGUIGNON ET AL: "Neuronal network coherent with hand kinematics during fast repetitive hand movements", NEUROIMAGE, ACADEMIC PRESS, ORLANDO, FL, US, vol. 59, no. 2, 12 septembre 2011 (2011-09-12), pages 1684-1691, XP028393028, ISSN: 1053-8119, DOI: 10.1016/J.NEUROIMAGE.2011.09.022 [extrait le 2011-09-22]
• MOHSENI H R ET AL: "Fusion of Magnetometer and Gradiometer Sensors of MEG in the Presence of Multiplicative Error", IEEE TRANSACTIONS ON BIOMEDICAL ENGINEERING, IEEE SERVICE CENTER, PISCATAWAY, NJ, USA, vol. 59, no. 7, 17 avril 2012 (2012-04-17), pages 1951-1961, XP011490126, ISSN: 0018-9294, DOI: 10.1109/TBME.2012.2195001 [extrait le 2012-04-17]
• Alexandre Barachant: "Filtrage spatial robuste à partir d'un sous-ensemble optimal d'électrodes en BCI EEG", Actes du Colloque GRETSI 2009, 8-11 septembre 2009, Dijon, France, 8 septembre 2009 (2009-09-08), XP055002259, Extrait de l'Internet: URL:http://documents.irevues.inist.fr/bits tream/handle/2042/28933/barachant_554.pdf? sequence=1 [extrait le 2011-07-07]
• CINCOTTI ET AL: "High-resolution EEG techniques for brain-computer interface applications", JOURNAL OF NEUROSCIENCE METHODS, ELSEVIER SCIENCE PUBLISHER B.V., AMSTERDAM, NL, vol. 167, no. 1, 29 novembre 2007 (2007-11-29), pages 31-42, XP022369303, ISSN: 0165-0270, DOI: 10.1016/J.JNEUMETH.2007.06.031

- **GROSS J ET AL: "DYNAMIC IMAGING OF COHERENT SOURCES: STUDYING NEURAL INTERACTIONS IN THE HUMAN BRAIN", PROCEEDINGS OF THE NATIONAL ACADEMY OF SCIENCES, NATIONAL ACADEMY OF SCIENCES, US, vol. 98, no. 2, 16 janvier 2001 (2001-01-16), pages 694-699, XP008047944, ISSN: 0027-8424, DOI: 10.1073/PNAS.98.2.694**
- **ELISEYEV A ET AL: "Classification of multi-modal data in a self-paced binary BCI in freely moving animals", ENGINEERING IN MEDICINE AND BIOLOGY SOCIETY,EMBC, 2011 ANNUAL INTERNATIONAL CONFERENCE OF THE IEEE, IEEE, 30 août 2011 (2011-08-30), pages 7147-7150, XP032320373, DOI: 10.1109/IEMBS.2011.6091806 ISBN: 978-1-4244-4121-1**
- **HENSON R N ET AL: "MEG and EEG data fusion: Simultaneous localisation of face-evoked responses", NEUROIMAGE, ACADEMIC PRESS, ORLANDO, FL, US, vol. 47, no. 2, 15 août 2009 (2009-08-15), pages 581-589, XP026436411, ISSN: 1053-8119 [extrait le 2009-05-03]**

**Description**

L'invention porte sur un procédé de localisation de l'activité cérébrale d'un sujet, notamment par magnétoencéphalographie. L'invention s'applique en particulier au domaine de la commande neuronale directe. L'invention est telle que divulguée dans les revendications.

La commande neuronale directe (BCI, de l'anglais « *brain-computer interface* », ou interface cerveau-ordinateur) permet d'établir une communication entre un utilisateur et une machine (typiquement un ordinateur) à travers des signaux neuronaux issus de l'activité cérébrale d'un sujet sans recourir à la voie musculaire, ce qui constitue un réel espoir pour les personnes souffrant de graves paralysies.

Les systèmes non intrusifs de commande neuronale directe utilisent, le plus souvent, l'électroencéphalographie (EEG) comme méthode d'acquisition de l'activité cérébrale. On place ainsi un certain nombre d'électrodes à la surface du crâne dans le but d'y mesurer une activité électrique reflétant l'activité cérébrale du sujet. D'autres techniques, plus performantes mais aussi plus intrusives, exploitent des signaux électrocorticographiques (ECoG), prélevés à la surface du cortex, voire des signaux prélevés par des électrodes profondes.

La magnétoencéphalographie (MEG) est une technique non intrusive, dont l'utilisation en commande neuronale directe est conceptuellement intéressante, car les signaux magnétiques ne subissent pas - ou peu - de distorsion lorsqu'ils se propagent à travers le crâne. A ce propos, on pourra se rapporter à l'article de J. Mellinger et al « An MEG-based Brain-Computer Interface (BCI) », Neuroimage 36(3) : 581 - 593 (1er juillet 2007). Le principal inconvénient de cette technique, qui en pratique la limite à des applications expérimentales, est l'insuffisante miniaturisation des capteurs magnétoencéphalographiques.

Quelle que soit la méthode d'acquisition de l'activité cérébrale utilisée, le principe à la base de la commande neuronale directe consiste généralement à associer une ou plusieurs tâches mentales (action imaginées par le sujet) à une ou plusieurs actions réalisées par un effecteur. Par exemple, l'imagination du mouvement de la main droite peut être associée au déplacement vers la droite d'un curseur.

La prise en compte de l'information spatiale véhiculée par les signaux neuronaux est importante pour réaliser cette association. En effet, l'effectuation de tâches mentales différentes active différentes régions du cerveau, ou les mêmes régions mais d'une manière différente. Pour préserver au maximum cette information spatiale on utilise, dans la plupart des cas, un grand nombre de capteurs (jusqu'à une centaine). Cette approche présente plusieurs inconvénients : une gêne pour l'utilisateur, un temps de préparation long, un coût calculatoire élevé. En outre, certains types de traitements montrent des limitations quand le nombre de capteurs augmente (par exemple, on observe des effets de sur-apprentissage).

Ainsi, des techniques ont été développées pour déterminer les emplacements optimaux, sur le crâne ou à la surface du cortex d'un sujet, où situer un nombre aussi limité que possible de capteurs. Par exemple, l'article de A. Barachant, T. Aksenova, et S. Bonnet, « Filtrage spatial robuste à partir d'un sous-ensemble optimal d'électrodes en BCI EEG » GRETSI 2009, 8 - 11 septembre 2009, décrit une méthode de sélection ascendante (c'est-à-dire dans laquelle on construit progressivement un ensemble optimal de capteurs), basée sur un critère de corrélation multiple de la log-variance des signaux EEG après filtrage fréquentiel.

L'article de F. Tadel et al. « Brainstorm : A User-Friendly Application for MEG/EEG Analysis », Computational Intelligence and Neuroscience, Vol. 2011, Article ID 879716, (2011) décrit un logiciel d'analyse de signaux électroencéphalographiques et magnétoencéphalographiques, permettant notamment leur représentation sous la forme de mappes temps-fréquence. Cette technique n'exploite que de manière incomplète l'information fournie par les capteurs magnétoencéphalographiques. En effet, un tel capteur comprend généralement trois capteurs élémentaires ou plus, et notamment :

- un magnétomètre, qui mesure l'intensité du champ magnétique en un point ; et
- deux gradiomètres planaires, perpendiculaires entre eux, qui mesurent deux composantes du gradient dudit champ magnétique.

Toutefois, pour la détermination des régions cérébrales qui s'activent dans une expérience de BCI, on n'utilise généralement qu'un seul signal représentatif du module du gradient du champ magnétique - sans tenir compte de sa direction, ou de l'intensité du champ.

Bourguignon et al. dans "Neuronal network coherent with hand kinematics during fast repetitive hand movements", NeuroImage 59 (2012), 1684-1691, ont quantifié le couplage entre des signaux corticaux magnetoencéphalographiques et des mouvements de main volontaires, répétitifs et rapides enregistrés par un accéléromètre à 3 axes.

Le présent texte vise à procurer un procédé de localisation de l'activité cérébrale d'un sujet permettant une meilleure exploitation de l'information acquise par les différents capteurs.

Un tel but est atteint par un procédé de localisation d'une activité cérébrale, comportant les étapes consistant à :

a) acquérir des données indicatives de stimuli sensoriels adressés à, ou d'actions volontaires performées ou imaginées par, un sujet ;

b) au moyen d'une pluralité de capteurs, acquérir des signaux représentatifs d'une activité, associée auxdits stimuli ou actions volontaires, de régions respectives du cerveau dudit sujet; et

c) pour chaque dit capteur, quantifier une corrélation existante entre lesdites données indicatives de stimuli sensoriels ou d'actions volontaires et les signaux acquis ;

caractérisé en ce que chaque dit capteur acquiert N≥2 signaux représentatifs de N grandeurs physiques distinctes, mesurées pour une même région du cerveau, et en ce que et ladite corrélation est établie entre une variable, et notamment une variable scalaire indicative d'un dit stimulus sensoriel ou d'une dite action volontaire et une variable de N ou d'au moins N dimensions représentative desdits signaux.

Autrement dit, on acquiert N signaux distincts, N≥2, en une même région du cerveau, et on établit une corrélation entre une variable représentative d'une action imaginée ou réalisée par un sujet, et une variable comportant au moins N composantes, chaque composante dépendant d'un des N signaux mesurés.

En particulier, on établit une corrélation entre une variable représentative d'une action imaginée ou réalisée par un sujet, et une variable comportant au moins N composantes, chaque composante ne dépendant que d'un des N signaux mesurés.

Ladite variable comportant au moins N composantes peut notamment comporter des valeurs des N signaux mesurés en une même région du cerveau, par un capteur ou un groupe de capteurs, ces signaux étant temporellement décalés par rapport à la variable représentative de l'action imaginée ou réalisée par le sujet.

Par même région du cerveau, on entend une zone spatiale inscrite dans un cercle de diamètre inférieur à 1cm, voire inférieur 0.5 cm, voire inférieure à 0.1 cm.

Avantageusement, ladite corrélation existante entre lesdites données indicatives de stimuli sensoriels ou d'actions volontaires et les signaux acquis par chaque dit capteur peut être quantifiée au moyen d'un coefficient de détermination.

A titre de comparaison, notamment lorsqu'on utilise des moyens de mesure magnétoencéphalographiques, les procédés connus de l'art antérieur et exploitant des mappes temps-fréquence sont intrinsèquement limitées à la prise en compte de signaux de type scalaire (par exemple : mesure du module du gradient du champ magnétique ou de l'intensité du champ magnétique).

En particulier, lesdits capteurs peuvent être des capteurs magnétoencéphalographiques. Dans ce cas, chaque dit capteur magnétoencéphalographique peut acquérir au moins un signal représentatif d'une intensité de champ magnétique et un signal représentatif d'une composante d'un gradient dudit champ magnétique ; en variante ou en complément, chaque dit capteur magnétoencéphalographique peut acquérir au moins deux signaux représentatifs de deux composantes d'un gradient dudit champ magnétique. Ainsi, la notion de capteur est à prendre au sens large ; au sens de l'invention, un capteur est apte à acquérir une pluralité de signaux en un même point ou en une même zone spatiale.

Ladite variable à N dimensions peut être obtenue en sélectionnant au moins une composante spectrale de chacun desdits signaux, acquis sur une fenêtre temporelle associée à un dit stimulus sensoriel ou action volontaire.

Selon un autre mode de réalisation ladite variable multidimensionnelle peut être obtenue en sélectionnant une pluralité de composantes spectrales d'un signal acquis sur une fenêtre temporelle associée à un dit stimulus sensoriel ou action volontaire.

Ces modes de réalisation peuvent être combinés : ainsi, ladite variable multidimensionnelle peut comprendre plusieurs composantes spectrales de plusieurs signaux représentatifs de grandeurs physiques respectives.

Le procédé peut comporter également une étape de visualisation dans laquelle on projette des valeurs indicatives de ladite corrélation, déterminées pour chaque dit capteur, sur un modèle tridimensionnel d'une surface corticale, et on réaliser une interpolation desdites valeurs entre différents points d'un maillage de ladite surface.

Un autre objet de la présente divulgation est un procédé de localisation de capteurs d'activité cérébrale pour commande neuronale directe comportant :

- une étape de localisation d'une activité cérébrale, mise en œuvre par un procédé tel que décrit ci-dessus ; et

- une étape de détermination de localisations optimales desdits capteurs d'activité cérébrale en fonction des résultats de ladite étape de localisation d'une activité cérébrale.

D'autres caractéristiques, détails et avantages de l'invention ressortiront à la lecture de la description faite en référence aux dessins annexés, donnés à titre d'exemple et qui représentent, respectivement :

- La figure 1, un casque pour magnétoencéphalographie utilisé pour la mise en œuvre d'un procédé selon la divulgation ;

- Les figures 2A, 2B et 2C, trois mappes bidimensionnelles des coefficients de corrélation entre les signaux acquis par trois capteurs magnétoencéphalographiques élémentaires et une variable indicative d'un stimulus visuel ;

- La figure 3, une mappe bidimensionnelle d'un coefficient de détermination généralisé combinant les informations contenues dans les mappes des figures 2B et 2C ;
- Les figures 4A et 4B, des mappes de localisation d'une activité cérébrale obtenues par un procédé équivalent à l'art antérieur et par un procédé selon un mode de réalisation de la divulgation respectivement ;
- Les figures 5A, 5B et 5C, des mappes de localisation d'une activité cérébrale obtenues par des procédés selon un autre mode de réalisation de la divulgation ; et
- La figure 6, une carte représentant une interpolation de la racine carrée de coefficients de détermination, projetée sur une représentation en trois dimension de la surface corticale.

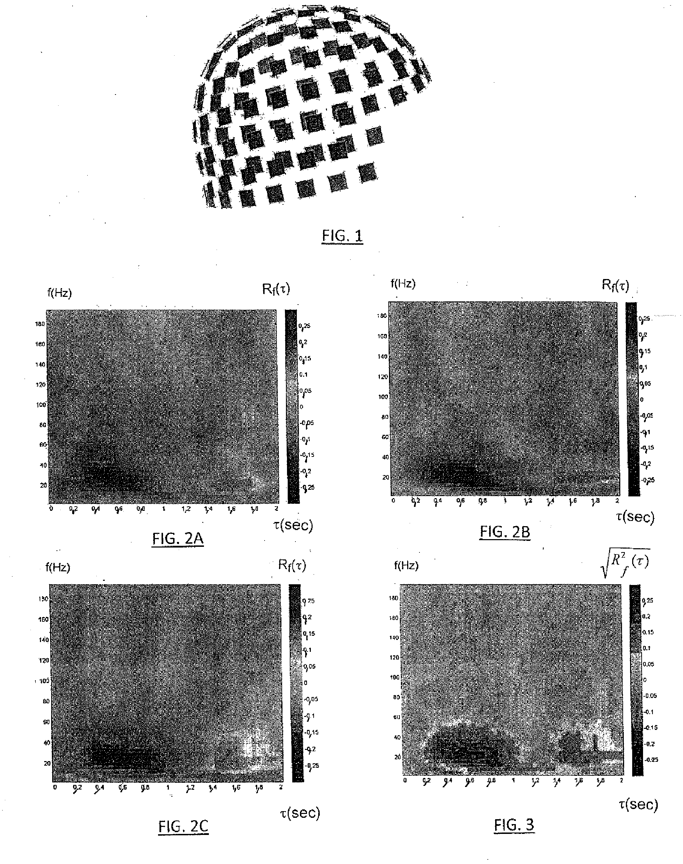

Les signaux MEG sont généralement acquis dans une chambre munie d'un blindage magnétique, au moyen d'un « casque MEG » placé sur le crâne du sujet, à une distance de 3 - 5 cm de la surface du cerveau. Le casque de la figure 1 se compose de 102 capteurs, ou « plaques MEG », comportant chacun trois capteurs élémentaires : un magnétomètre et deux gradiomètres planaires du 1$^{er}$ ordre présentant des axes de sensibilité orthogonaux et gisant dans le plan de la plaque, pour un total de 306 capteurs élémentaires. Ces capteurs permettent de caractériser les courants électriques d'orientation tangentielle générés dans les sulci du cerveau. Il existe également d'autres types de capteurs MEG, tels que les gradiomètres radiaux, mais leur utilisation est moins fréquente. Pour plus d'informations à ce sujet on pourra se référer à l'article de J. Vrba et S. E. Robinson « Signal Processing in Magnetoencephalography », Methods 25, 249 - 271 (2001).

Les mouvements de la tête du sujet sont enregistrés et compensés au moyen de bobines placées à des points cardinaux prédéfinis (nasion, canaux des oreilles) et générant des champs magnétiques alternatifs à une fréquence éloignée de celle des signaux MEG d'intérêt.

Dans une mise en œuvre expérimentale du procédé de la divulgation le sujet a reçu la consigne d'effectuer ou imaginer un mouvement de flexion/extension de l'index gauche en réponse à un stimulus visuel. Les signaux acquis par les 306 capteurs élémentaires du casque de la figure 1 ont été échantillonnés à une fréquence de 1000 Hz et filtrés pour ne retenir que la bande 0 - 200 Hz. Puis, ils ont subi une décomposition temps-fréquence par transformation en ondelettes continue. Ainsi, chaque signal $s^i(t)$ (i=1 - 306 étant l'indice permettant de repérer le capteur élémentaire dont le signal est issu) est représenté par une mappe temps-fréquence $x^i_f(t)=CWT_f[s^i(t)]$, où CWT est l'opérateur de transformation par ondelettes continue et « f » est la fréquence. La transformation en ondelettes peut être suivie d'un procédé de lissage et de normalisation. Par exemple, on peut déterminer la valeur absolue de $CWT_f[s^i(t)]$, puis appliquer une moyenne glissante, par exemple selon une durée de 300 ms, ce qui peut s'écrire : $Liss(abs(CWT_f[s^i(t)]))$, où « *Liss* » est l'opérateur de lissage et « *abs* » l'opérateur de valeur absolue. Chaque composante fréquentielle de cette grandeur peut ensuite être normalisée par exemple par sa variance, ce qui permet d'établir un équilibre entre les valeurs à basse fréquence, généralement plus intenses que les valeurs à haute fréquence.

Il est possible de calculer un coefficient de corrélation de Pearson entre chaque composante fréquentielle $x^i_f(t)$ d'un signal élémentaire $s^i(t)$ et une variable binaire y(t) indicative d'un stimulus visuel. Par exemple, y(t) peut prendre la valeur 1 si le stimulus est émis au temps t, et 0 dans le cas contraire. Le coefficient de corrélation de Pearson décalé à la fréquence f, $R_f$, est donné par :

$$R_f(\tau) = \frac{\frac{1}{n}\sum x_f(t)\left(y(t-\tau)-\overline{y}\right)}{\sigma_{x_f}\sigma_y} \qquad [1]$$

où :

- $\overline{y}$ est la valeur moyenne de y(t) (on supposé que $x^i_f(t)$ est centrée, c'est-à-dire que $\overline{x_f}$ =0, la généralisation au cas d'une variable non centrée est banale) ;
- $\sigma_x$ et $\sigma_y$ sont les variances de $x^i_f(t)$ et y(t), respectivement ;
- $\tau$ est le délai écoulé depuis le stimulus, compris, par exemple, entre 0 et 2 secondes, ce qui signifie qu'on s'intéresse à la réponse du cerveau dans les 2 secondes qui suivent l'émission d'un stimulus ;
- n est le nombre d'échantillons temporels.

Les figures 2A - 2C montrent des graphiques de $R_f(\tau)$ en fonction de la fréquence f et du délai $\tau$ pour trois capteurs élémentaires (un magnétomètre - figure 2A - et deux gradiomètres orthogonaux - figures 2B et 2C), identifiés par i=120, 121 et 122 respectivement, portés par un même capteur MEG. Ces graphiques montrent que le coefficient de corrélation prend une valeur sensiblement différente de zéro pour les composantes spectrales dans la plage 10 - 40 Hz, et que ce coefficient est négatif pour $\tau$ compris entre 0,2 et 1 sec. et positif pour $\tau$ compris entre 1,4 et 2 sec. ; la valeur maximale de $|R_f(\tau)|$ pour le magnétomètre 120 et les gradiomètres 121 et 122 vaut respectivement 0,17, 0,19 et 0,18 (la valeur maximale possible étant de 1). Cela indique qu'il serait possible, par exemple, de réaliser une commande neuronale directe en utilisant les composantes spectrales comprises entre 20 et 30 Hz du signal $s^{121}(t)$ dans l'intervalle 0,4 - 0,8 secondes après l'émission d'un stimulus. En comparant entre eux les graphiques associés à différents capteurs MEG, en outre, il est possible de sélectionner les mieux adaptés pour une application de type BCI. Comme chaque capteur est sensible aux courants électriques qui circulent dans les régions du cerveau immédiatement adjacentes, ces graphiques permettent aussi de localiser l'activité cérébrale du sujet en réponse au stimulus.

Plus que la valeur algébrique du coefficient $R_f(\tau)$, c'est sa valeur absolue qui présente un intérêt direct pour la localisation de l'activité cérébrale et, par conséquent, pour la détermination des endroits optimaux où peuvent être positionnés de capteurs pour applications BCI. Par conséquent, on peut considérer, au lieu de $R_f(\tau)$, son carré $R^2_f(\tau)$, appelé coefficient de détermination, ou la racine carrée de ce dernier.

On peut montrer que, à la condition (généralement vérifiée) que y(t) prenne la valeur 0 la plupart du temps - c'est-à-dire à la condition que les stimuli soient relativement courts et espacés entre eux - l'information contenue dans les graphiques des figures 2A - 2C est équivalente à celle véhiculée par les mappes temps-fréquence, utilisées dans l'article précité de Tadel et al. Toutefois, contrairement aux mappes temps-fréquence, le coefficient de détermination $R^2_f(\tau)$ peut être généralisé au cas d'une variable vectorielle. Par exemple, on peut considérer la variable $x_f(t)=[x^1_f(t) \ldots x^N_f(t)]T$ (l'exposant « T » indique l'opération de transposition), dont chaque élément est une composante spectrale d'un capteur élémentaire (donc N=2 si l'on considère les signaux issus de 2 gradiomètres).

En effet, le coefficient de détermination estime la fraction de la variance de y(t) qui est expliquée par $x_f(t)$ au moyen d'une régression (multi)linéaire :

$$R_f^{\ 2}(\tau) = 1 - \frac{\sum \left( y(t) - \hat{y}(t) \right)^2}{\sum \left( y(t) - \bar{y} \right)^2} \qquad [2]$$

avec

$$\hat{y}(t) = a + \sum_{i=1}^{N} b_i x_f^i(t + \tau) \qquad [3]$$

les coefficients a et $b_i$ de l'équation [3] étant calculés par la méthode des moindres carrés.

D'une façon générale, ce coefficient de détermination exprime la corrélation entre y(t) et l'estimation de y(t), notée $\hat{y}(t)$, à l'aide de la variable $X_f(t+\tau)$.

La figure 3 est un graphique bidimensionnel de $\sqrt{R_f^2(\tau)}$, obtenu en considérant la variable $x_f(t+\tau)=[x^{121}_f(t+\tau), x^{122}_f(t+\tau)]^T$ qui combine les signaux issus des deux gradiomètres 121, 122.

Plus précisément, à partir de cette variable, il est possible de constituer une matrice

$$\mathbf{X} = \begin{pmatrix} 1 & x_f^1(t_1 + \tau) & x_f^2(t_1 + \tau) \\ 1 & x_f^1(t_2 + \tau) & x_f^2(t_2 + \tau) \\ \ldots & \ldots & \ldots \\ 1 & x_f^1(t_n + \tau) & x_f^2(t_n + \tau) \end{pmatrix}, \ x_f^i(t_i + \tau)$$

représentant la composante spectrale dans la bande de fréquence f du gradiomètre i mesurée à l'instant $t+\tau$ suivant l'instant $t_j$ d'échantillonnage de la variable y(t). Le décalage temporel, noté $\tau_1$ est généralement supérieur à 0s et inférieur à 5s, de préférence inférieur à 2s, et cela pour l'ensemble des modes de réalisation.

On constitue également un vecteur y rassemblant les différentes valeurs $y(t_j)$, tel que $y = (y(t_1) \ y(t_2) \ldots y(t_N))^T$.

On détermine ensuite $\hat{y}(t) = b_0 + b_1 x_f^1(t+\tau) + b_2 x_f^2(t+\tau)$, $b_0$, $b_1$ et $b_2$ étant estimés selon la méthode des moindre carrés, par exemple selon

**b = (X$^T$X)$^{-1}$X$^T$y**, avec b = ($b_0$ $b_1$ $b_2$)$^T$

Le coefficient de détermination est ensuite calculé en appliquant l'équation [2].

Ainsi, selon ce mode de réalisation,

- on utilise un nombre N de signaux N étant ici égal à 2, mesurés en un même point (ou au moins en une même région du cerveau au sens de l'invention.).
- on détermine la composante fréquentielle de chaque signal à la fréquence f, ce qui permet de constituer une variable $x_f(t+\tau)$, chaque terme de la variable $x_f(t+\tau)$ comprenant la composante fréquentielle, à la fréquence f, notée $x^k_f(t+\tau)$ d'un signal $x^k$ mesuré par le gradiomètre k à un instant t + $\tau$. La dimension de cette variable $x_f(t+\tau)$ est N.
- on établit une corrélation entre une variable représentative d'une action imaginée ou réalisée par un sujet (en l'occurrence , y(t)) et la variable $x_f(t+\tau)$ à N dimensions.

On note que dans cet exemple, chaque composante de la variable $x_f(t+\tau)$ est déterminée à l'aide d'un seul des N signaux mesurés pour ladite région du cerveau. Par ailleurs, la variable $\tau$ représente le décalage temporel entre les signaux $x^k$ considérés et l'estimation de la variable y(t).

La valeur maximale de $\sqrt{R_f^2(\tau)}$ est de 0,21, à comparer à une valeur de 0,19 obtenue en prenant en considération un seul gradiomètre. Ainsi, le fait de combiner des signaux différents, enregistrés au même endroit, permet d'augmenter la sensibilité de la détection d'une modification de l'activité cérébrale en réponse à un stimulus.

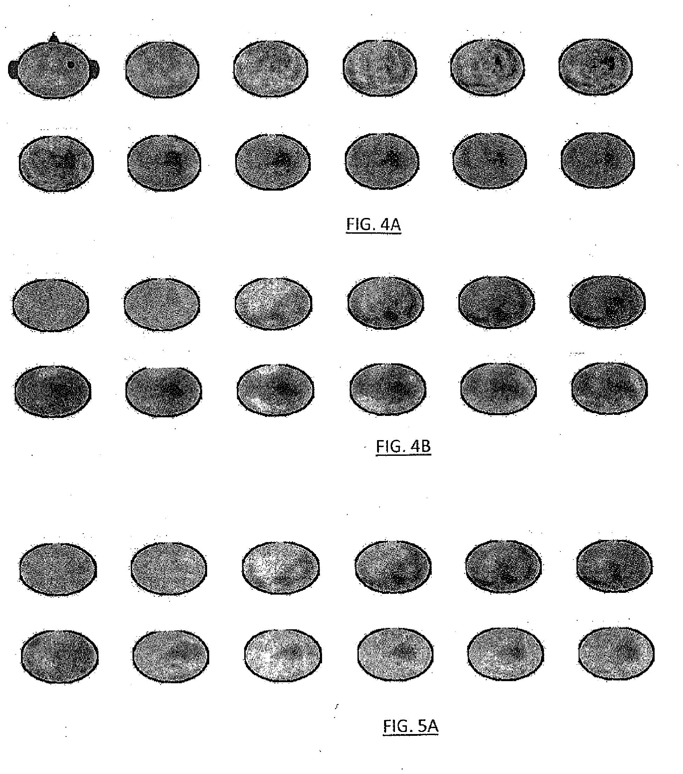

Les figures 4A et 4B montrent des mappes spatiales de l'activité cérébrale (mouvement imaginé de l'index gauche) d'un sujet en réponse à un stimulus visuel, à des instants différents après l'émission dudit stimulus. Plus précisément :

- Les mappes de la figure 4A montrent les coefficients de corrélation R($\tau$) entre les signaux issus d'un gradiomètre pour chaque capteur MEG et la variable binaire y(t) ; le délai $\tau$ est compris entre 0,00 sec. pour la mappe de gauche de la première ligne et 0,88 sec. pour la mappe de droite de la seconde ligne, avec des incréments de 0,08 sec. entre chaque image.

Cette figure montre, pour un gradiomètre de chaque capteur, la valeur du coefficient de corrélation ayant la plus forte valeur absolue, que le coefficient soit positif ou négatif. Autrement dit, cette figure représente

$$\mathrm{extremum}_f\left[R_f(\tau)\right],$$

où « *extrémum* » est l'opérateur qui associe à la fonction qui en constitue l'argument sa valeur extrémale (maximale ou minimale), ce qui, dans la pratique, équivaut à $\max_f \sqrt{R_f^2(\tau)}$, les corrélations négatives ayant généralement une faible valeur absolue

Le cercle sur la première mappe de la première ligne montre la localisation des capteurs élémentaires 120 - 122 mentionnés plus haut.

- Les mappes de la figure 4B montrent les racines carrées des coefficients de détermination maximaux

$$\max_f \sqrt{R_f^2(\tau)}$$

calculés en prenant en considération les signaux issus des deux gradiomètres de chaque capteur MEG. Comme pour la figure 4A, le délai $\tau$ est compris entre 0,00 sec. et 0,68 sec. avec des incréments de 0,08 sec. entre chaque image, et les coefficients sont intégrés entre 5 et 200 Hz.

On peut noter que l'analyse multicanaux (figure 4B) permet une meilleure localisation de l'activité cérébrale. D'un point de vue quantitatif, cela est exprimé par le fait que, dans le cas de l'analyse monocanal de la figure 4A, |R($\tau$)| prend une valeur maximale de 0,2 tandis que dans le cas de la figure 4B la valeur maximale de $\sqrt{R^2(\tau)}$ atteint 0,3.

L'utilisation des coefficients de détermination généralisés permet également une meilleure exploitation de l'information fréquentielle. Ainsi, il est possible d'utiliser une variable x (t)=[ $x^1_{f1}(t)$... $x^1_{fM}(t)$ ... $x^N_{f1}(t)$... $x^{Nc}_{fM}(t)$]$^T$. Cette variable combine les signaux de N capteurs élémentaires en prenant en considération M composantes spectrales (indices f1 - fM) pour chacun d'entre eux. Sa dimension est N·M.

Plus précisément, si $t_j$ représente l'instant auquel on acquiert la valeur de y(t), on utilise la variable x ($t_i+\tau$)=[ $x^1_{f1}(t_i$

$+\tau$)... $x^1_{fM}(t_i + \tau)$ ... $x^{Nc}_{f1}(t_i+\tau)$... $x^{Nc}_{fM}(t_i+\tau)]^T$, $\tau$ étant l'intervalle temporel séparant la mesure de cet instant $t_i$. On peut établir, à chaque intervalle $\tau$, un coefficient de régression $R(\tau)$ en constituant la matrice X, dont chaque ligne comporte la variable x $(t_i+\tau)$ :

$$\mathbf{X} = \begin{pmatrix} 1 & x^1_{f1}(t_1+\tau) & x^1_{f2}(t_1+\tau) & ... & x^2_{f1}(t_1+\tau) & x^2_{f2}(t_1+\tau) & ... \\ 1 & x^1_{f1}(t_2+\tau) & x^1_{f2}(t_2+\tau) & ... & x^2_{f1}(t_2+\tau) & x^2_{f2}(t_2+\tau) & ... \\ ... & ... & ... & ... & ... & ... & ... \\ 1 & x^1_{fM}(t_N+\tau) & x^1_{fM}(t_N+\tau) & ... & x^2_{fM}(t_N+\tau) & x^2_{fM}(t_N+\tau)... \end{pmatrix}$$

et le vecteur d'observations y = $(y(t_1)\, y(t_2) ... y(t_N))^T$.

Dans l'expression de la matrice X, $x^i_{f_k}(t_j+\tau)$ représente la composante spectrale dans la bande de fréquence $f_k$ du gradiomètre i mesurée à l'instant $t+\tau$ suivant l'instant $t_j$ d'échantillonnage de la variable y(t).

Le coefficient de régression $R(\tau)$ est obtenu en déterminant

$$\hat{y}\,(t) = b_0 + \sum_{i=1}^{M} b^1_i\, x^{1'}_{fi}(t+\tau) + \sum_{i=1}^{M} b^2_i\, x^2_{fi}(t+\tau)$$

et :

$$\mathbf{b} = \begin{pmatrix} b_0 & b^1_1 & b^1_2 ... & b^2_i & b^2_2 & ... & b^2_M \end{pmatrix}^T$$

tel que b = $(\mathbf{X}^T\mathbf{X})^{-1}\mathbf{X}^T\mathbf{y}$.

Ainsi, selon ce mode de réalisation,

- on utilise un nombre N de signaux, N étant ici égal à 2, mesurés en un même point (ou une même région du cerveau au sens de l'invention)
- on détermine la composante fréquentielle de chaque signal à différentes fréquence $f_1...f_M$, ce qui permet de constituer une variable x $(t+\tau)=[\,x^1_{f1}(t+\tau)... x^1_{fM}(t+\tau) ... x^N_{f1}(t+\tau)... X^N_{fM}(t+\tau)]^T$, où chaque terme de la variable x$(t+\tau)$ comprend la composante fréquentielle, à la fréquence $f_i$, notée $x^k_{fi}(t+\tau)$ d'un signal $x^k$ mesuré à un instant $t + \tau$ par le capteur k. La dimension de cette variable $x_f(t+\tau)$ est N * M, N étant le nombre de signaux adressant la même région du cerveau et M étant le nombre de composantes fréquentielles,
- on établit une corrélation entre une variable représentative d'une action imaginée ou réalisée par un sujet (en l'occurrence , y(t)) et la variable x$(t+\tau)$ à N dimensions.

On note que dans cet exemple, chaque composante de la variable x$(t+\tau)$ est déterminée à l'aide d'un seul des N signaux mesurés pour ladite région du cerveau. Par ailleurs, la variable $\tau$ représente le décalage temporel entre les signaux $x^k$ considérés et la variable y(t)

Les figures 5A - 5C des mappes d'activité cérébrale obtenues dans des conditions similaires à celles de la figure 4B, en combinant une ou plusieurs composantes spectrales de signaux issus de deux gradiomètres pour chaque capteur MEG. Toutefois :

- Dans le cas de la figure 5A, une seule composante spectrale dans la région 5 - 12 Hz a été considérée : Nc=2, M=1, N=2. La valeur maximale de $\sqrt{R^2(\tau)}$ s'élève à 0,14.

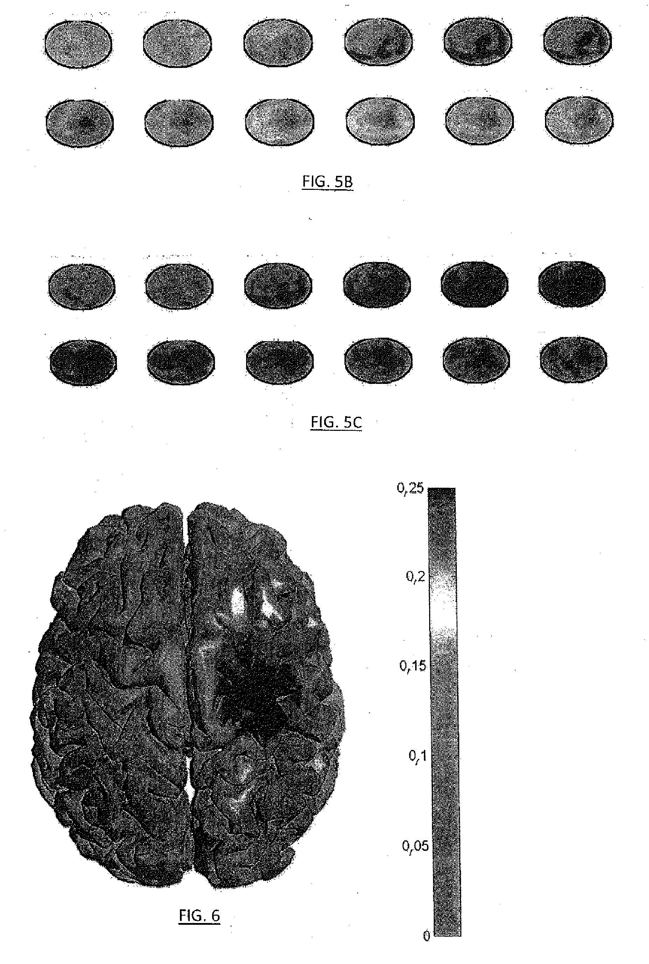

- Dans le cas de la figure 5B, trois composantes spectrales dans la région 5 - 12 Hz ont été considérées: Nc=2, M=3, N=6. La valeur maximale de $\sqrt{R^2(\tau)}$ s'élève à 0.2

- Dans le cas de la figure 5C, 21 composantes spectrales dans la région 5 - 200 Hz ont été considérées : Nc=2, M=21, N=42. La valeur maximale de $\sqrt{R^2(\tau)}$ s'élève à 0.35

La valeur maximale de $\sqrt{R^2(\tau)}$ augmente avec le nombre M de composantes spectrales considérées pour l'analyse. Toutefois, dans le cas de la figure 5C on observe une diminution du contraste, qui nuit à la localisation de l'activité cérébrale. Il s'agit là d'un problème de sur-apprentissage, auquel on peut remédier par des techniques connues.

Une technique possible est d'appliquer un algorithme de validation croisée, en divisant l'ensemble des signaux mesurés en Q sous-ensembles. On constitue alors Q sous-matrices $X_q$, chacune correspondant à un vecteur $y_q$

Chaque terme d'un sous-vecteur $\hat{y}_q$ est alors estimé selon la relation précédente, le vecteur

$$\mathbf{b}^q = \begin{pmatrix} b_0^q & b_1^{1q} & b_2^{1q} \dots & b_1^{2q} & b_2^{2q} & \dots & b_M^{2\,q} \end{pmatrix}^T$$

étant déterminé en utilisant les sous-matrices $X_p$, avec $p \neq q$.

De plus, on peut mettre en œuvre un algorithme de régression de Ridge, qui permet une détermination plus stable du vecteur b

Une nouvelle série d'essais a été réalisée. Le tableau ci-dessous représente un comparatif des valeurs maximales de la racine carrée du coefficient de détermination $\sqrt{R^2(\tau)}$, soit pour l'ensemble des capteurs, soit pour l'ensemble des fréquences et l'ensemble des capteurs, ce coefficient étant obtenu en réalisant différentes actions lors de l'observation d'un stimulus :

- 1ère ligne : mouvement imaginaire de l'index gauche
- 2ième ligne : mouvement réel de l'index gauche
- 3ième ligne : mouvement imaginaire de l'index droit
- 4ième ligne : mouvement réel de l'index droit.

La première colonne du tableau indique les valeurs maximales, pour l'ensemble des fréquences considérées, de la racine carrée du coefficient de détermination, en utilisant des valeurs issues d'un seul gradiomètre, c'est à dire $\max_{f,\tau} \sqrt{\left(R_{n,f}(\tau)\right)^2}$, où $R_{n,f}(\tau)$ représente le coefficient de corrélation obtenu en utilisant la composante spectrale à la fréquence f mesurée par le gradiomètre n à l'instant $\tau$.

La deuxième colonne du tableau indique les valeurs maximales de la racine carrée du coefficient de détermination, pour l'ensemble des fréquences considérées, en utilisant le module de la somme des signaux produits, en un même point, par deux gradiomètres c'est à dire $\max_{f,\tau} \sqrt{\left(R_f(\tau)\right)^2}$ où $R_f(\tau)$ représente le coefficient de corrélation obtenu en utilisant la composante spectrale à la fréquence f du module de la somme des signaux produits par deux gradiomètres orthogonaux situés au même point n à l'instant $\tau$.

La troisième colonne du tableau indique les valeurs maximales de la racine carrée du coefficient de détermination, en combinant les composantes spectrales en un même point, des signaux mesurés par deux gradiomètres. $\max_{\tau} \sqrt{\left(R_f(\tau)\right)^2}$ où $R(\tau)$ représente le coefficient de corrélation obtenu en utilisant les différentes composantes spectrales des signaux produits par deux gradiomètres orthogonaux situés au même point n à l'instant $\tau$. Les résultats représentés dans cette colonne ont été obtenus en combinant les signaux de 2 gradiomètres dans les 21 bandes de fréquence considérées.

|  | $\underset{f,\tau}{Max} \sqrt{(R_{n,f}(\tau))^2}$ | $\underset{f,\tau}{Max} \sqrt{(R_f(\tau))^2}$ | $\underset{\tau}{Max} \sqrt{(R(\tau))^2}$ |
|---|---|---|---|
| index gauche (imaginaire) | 0.192 | 0.206 | 0.3 |
| Index gauche (réel) | 0.252 | 0.363 | 0.461 |
| Index droit (imaginaire) | 0.234 | 0.228 | 0.292 |

(suite)

|  | $Max_{f,\tau} \sqrt{(R_{n,f}(\tau))^2}$ | $Max_{f,\tau} \sqrt{(R_f(\tau))^2}$ | $Max_{\tau} \sqrt{(R(\tau))^2}$ |
|---|---|---|---|
| Index droit (réel) | 0.375 | 0.374 | 0.470 |

Ces résultats expérimentaux montrent qu'on obtient un coefficient de détermination plus élevé lorsqu'on combine différentes composantes spectrales des signaux issus de plusieurs gradiomètres (en l'occurrence deux) disposés en un même point (3$^{ième}$ colonne).

Selon les différents modes de réalisation décrits ci-dessus, on aboutit à un ou plusieurs coefficients de corrélation par point de mesure en fonction de la durée $\tau$ entre le stimulus et la mesure. On dispose alors de valeurs de coefficients de corrélation (ou de détermination) selon un maillage spatial défini par le positionnement des capteurs. Il est possible de se baser sur ce maillage pour réaliser une projection desdites valeurs à la surface du cortex. Pour cela, la surface du cortex est obtenue, par exemple à partir de mesures IRM, puis est modélisée. Le maillage formé par les différents capteurs est ensuite recalé par rapport à ce modèle, par exemple en utilisant des repères stéréotaxiques visibles en IRM, notamment des pastilles en sel de gadolinium disposées sur la tête du patient.

A partir des valeurs de coefficients de détermination, on réalise une projection sur le modèle de la surface corticale, la valeur attribuée à chaque élément de ladite surface corticale étant issue d'une interpolation entre différents points du maillage, par exemple les trois plus proches voisins (S, S1, S2), le critère de pondération étant une distance. On obtient ainsi une image telle que la figure 6. Cette figure a été obtenue en interpolant les valeurs de racine carrée du coefficient de détermination à 0,48 s d'un stimulus, à la vue duquel le patient doit imaginer le mouvement de l'index gauche.

La divulgation a été décrite en référence à des modes de réalisation particuliers, utilisant des capteurs MEG comportant des magnétomètres et des gradiomètres planaires du premier ordre, et une variable binaire y représentative d'un stimulus visuel. Cependant, il ne s'agit nullement de limitations essentielles. En effet, le procédé de la divulgation peut être appliqué à des capteurs différents ; il permet aussi de combiner des signaux issus de capteurs de natures différentes - par exemple des magnétomètres et des électrodes d'EEG ou de ECoG. Par ailleurs, la variable y n'est pas nécessairement binaire, elle peut prendre plus généralement des valeurs discrètes ou continues.

**Revendications**

1. Procédé de localisation d'une activité cérébrale, comportant les étapes consistant à :

    a) acquérir des données indicatives de stimuli sensoriels adressés à, ou d'actions volontaires performées ou imaginées par, un sujet ;
    b) au moyen d'une pluralité de capteurs, acquérir des signaux représentatifs d'une activité, associée auxdits stimuli ou actions volontaires, de régions respectives du cerveau dudit sujet; et
    c) pour chaque dit capteur, quantifier une corrélation existante entre lesdites données indicatives de stimuli sensoriels ou d'actions volontaires et les signaux acquis ;

    chaque dit capteur acquérant N≥2 signaux représentatifs de N grandeurs physiques distinctes, mesurées pour une même région du cerveau, et ladite corrélation étant établie entre une variable (y) indicative d'un dit stimulus sensoriel ou d'une dite action volontaire et une variable (xf, x) d'au moins N dimensions représentative desdits signaux, **caractérisé en ce que** ladite corrélation est établie entre ladite variable indicative d'un dit stimulus sensoriel ou d'une dite action volontaire à l'instant t (y(t)) et ladite variable d'au moins N dimensions ($x_f(t+\tau)$, $x(t+\tau)$), représentative desdits signaux mesurés pour une même région du cerveau, lesdits signaux étant temporellement décalés par rapport audit instant t.

2. Procédé selon la revendication précédente, dans lequel ladite corrélation existante entre lesdites données indicatives de stimuli sensoriels ou d'actions volontaires et les signaux acquis par chaque dit capteur est quantifiée au moyen d'un coefficient de détermination.

3. Procédé selon l'une des revendications précédentes, dans lequel lesdits capteurs sont des capteurs magnétoencéphalographiques.

4. Procédé selon la revendication 3, dans lequel chaque dit capteur magnétoencéphalographique acquiert au moins un signal représentatif d'une intensité de champ magnétique et un signal représentatif d'une composante d'un gradient dudit champ magnétique.

5. Procédé selon l'une des revendications 3 et 4, dans lequel chaque dit capteur magnétoencéphalographique acquiert au moins deux signaux représentatifs de deux composantes d'un gradient dudit champ magnétique.

6. Procédé selon l'une des revendications précédentes, dans lequel ladite variable à N dimensions est obtenue en sélectionnant au moins une composante spectrale de chacun desdits signaux, acquis sur une fenêtre temporelle associée à un dit stimulus sensoriel ou action volontaire.

7. Procédé selon l'une des revendications précédentes, dans lequel ladite variable multidimensionnelle est obtenue en sélectionnant une pluralité de composantes spectrales d'un signal acquis sur une fenêtre temporelle associée à un dit stimulus sensoriel ou action volontaire.

8. Procédé selon l'une des revendications précédentes, comportant également une étape de visualisation dans laquelle on projette des valeurs indicatives de ladite corrélation, déterminées pour chaque dit capteur, sur un modèle tridimensionnel d'une surface corticale, et on réalise une interpolation desdites valeurs entre différents points d'un maillage de ladite surface.

9. Procédé de localisation de capteurs d'activité cérébrale pour commande neuronale directe comportant :

   - une étape de localisation d'une activité cérébrale, mise en œuvre par un procédé selon l'une des revendications précédentes ; et
   - une étape de détermination de localisations optimales desdits capteurs d'activité cérébrale en fonction des résultats de ladite étape de localisation d'une activité cérébrale.


**Patentansprüche**

1. Verfahren zur Lokalisierung einer Gehirnaktivität, das die Schritte umfasst, die bestehen aus:

   a) Erfassen der Daten, die auf Sinnesreize hinweisen, die auf ein Subjekt gerichtet sind, oder absichtlicher Aktionen, die dieses ausführt oder sich vorstellt;
   b) mittels einer Vielzahl von Sensoren, Erfassen der Signale, die für eine Aktivität, die mit den Reizen oder absichtlichen Aktionen verbunden sind, repräsentativ sind, aus jeweiligen Regionen des Gehirns des Subjekts; und
   c) für jeden Sensor Quantifizieren einer existierenden Korrelation zwischen den Daten, die auf Sinnesreize oder absichtliche Handlungen hinweisen, und den erfassten Signalen;

   wobei jeder Sensor N≥2 Signale erfasst, die für N getrennte physikalische Größen repräsentativ sind, die für dieselbe Region des Gehirns gemessen werden, und wobei die Korrelation zwischen einer Variablen (y), die auf einen Sinnesreiz oder eine absichtliche Handlung hinweist, und einer Variablen (xf, x) mit mindestens N Dimensionen, für die Signale repräsentativ ist, ermittelt wird,
   **dadurch gekennzeichnet, dass** die Korrelation zwischen der Variablen, die auf einen Sinnesreiz oder eine absichtliche Handlung in dem Augenblick t(y(t)) hinweist, und der Variablen mit mindestens N Dimensionen ($x_f(t+\tau)$, $x(t+\tau)$), die für die Signale repräsentativ ist, die für dieselbe Region des Gehirns gemessen werden, ermittelt wird, wobei die Signale zeitlich in Bezug auf den Augenblick t versetzt sind.

2. Verfahren nach dem vorstehenden Anspruch, wobei die existierende Korrelation zwischen den Daten, die auf Sinnesreize oder absichtliche Handlungen hinweisen, und den Signalen, die von dem Sensor erfasst werden, existiert, mittels eines Bestimmungskoeffizienten quantifiziert wird.

3. Verfahren nach einem der vorstehenden Ansprüche, wobei die Sensoren Magnetenzephalographiesensoren sind.

4. Verfahren nach Anspruch 3, wobei jeder Magnetenzephalographiesensor mindestens ein Signal, das für eine Magnetfeldstärke repräsentativ ist, und ein Signal, das für eine Komponente eines Gradienten des Magnetfelds repräsentativ ist, erfasst.

**5.** Verfahren nach einem der Ansprüche 3 und 4, wobei jeder Magnetenzephalographiesensor mindestens zwei Signale erfasst, die für zwei Komponenten eines Gradienten des Magnetfelds repräsentativ sind.

**6.** Verfahren nach einem der vorstehenden Ansprüche, wobei die Variable mit N Dimensionen erhalten wird, indem mindestens eine spektrale Komponente jedes der Signale, die in einem Zeitfenster erfasst werden, das mit einem Sinnesreiz oder einer absichtlichen Aktion verbunden ist, ausgewählt wird.

**7.** Verfahren nach einem der vorstehenden Ansprüche, wobei die multidimensionale Variable erhalten wird, indem mindestens eine Vielzahl spektraler Komponenten eines Signals, das in einem Zeitfenster erfasst wird, das mit einem Sinnesreiz oder einer absichtlichen Aktion verbunden ist, ausgewählt wird.

**8.** Verfahren nach einem der vorstehenden Ansprüche, das auch einen Visualisierungsschritt umfasst, bei dem Richtwerte der Korrelation, die für jeden Sensor bestimmt werden, auf ein dreidimensionales Modell einer kortikalen Oberfläche projiziert werden, und eine Interpolation der Werte zwischen unterschiedlichen Punkten eines Maschennetzes der Oberfläche ausgeführt wird.

**9.** Verfahren zur Lokalisierung von Gehirnaktivitätssensoren für direkte neuronale Steuerung, das umfasst:

- einen Lokalisierungsschritt einer Gehirnaktivität, der durch ein Verfahren nach einem der vorstehenden Ansprüche umgesetzt wird; und
- einen Bestimmungsschritt optimaler Lokalisierungen der Gehirnaktivitätssensoren in Abhängigkeit von den Resultaten des Lokalisierungsschritts einer Gehirnaktivität.

## Claims

**1.** Method for locating a brain activity, comprising the steps consisting of:

a) acquiring data indicative of sensory stimuli addressed to, or deliberate actions performed or imagined by, a subject;
b) by means of a plurality of sensors, acquiring signals representative of an activity, associated with said stimuli or deliberate actions, of respective regions of the brain of said subject; and
c) for each said sensor, quantifying a correlation that exists between said data indicative of sensory stimuli or of deliberate actions and the signals acquired;

each said sensor acquiring $N \geq 2$ signals that are representative of N distinct physical quantities, measured for the same region of the brain, and in that said correlation is established between a variable (y) indicative of a said sensory stimulus or of a said deliberate action and a variable (xf, x) of at least N dimensions representative of said signals, **characterised in that** said correlation is established between said variable indicative of a said sensory stimulus or of a said deliberate action and at the instant t (y(t)) and said variable of at least N dimensions ($x_f(t+\tau)$, $x(t+\tau)$), representative of said measured signals for the same region of the brain, said signals being offset in time relative to said instant t.

**2.** Method according to the preceding claim, wherein said correlation that exists between said data indicative of sensory stimuli or of deliberate actions and the signals acquired by each said sensor is quantified by means of a determination coefficient.

**3.** Method according to one of the preceding claims, wherein said sensors are magnetoencephalographic sensors.

**4.** Method according to claim 3, wherein each said magnetoencephalographic sensor acquires at least one signal representative of a magnetic field intensity and one signal representative of a component of a gradient of said magnetic field.

**5.** Method according to one of claims 3 and 4, wherein each said magnetoencephalographic sensor acquires at least two signals representative of two components of a gradient of said magnetic field.

**6.** Method according to one of the preceding claims, wherein said variable of at least N dimensions is obtained by selecting at least one spectral component of each of said signals, acquired over a time window associated with a said sensory stimulus or deliberate action.

7. Method according to one of the preceding claims, wherein said multidimensional variable is obtained by selecting a plurality of spectral components of a signal acquired over a time window associated with a said sensory stimulus or deliberate action.

8. Method according to one of the preceding claims, also comprising a visualisation step wherein values indicative of said correlation, determined for each said sensor, are projected onto a three-dimensional model of a cortical surface, and an interpolation of said values between different points of a mesh of said surface is performed.

9. Method for locating brain activity sensors for direct neural control comprising:

   - a step of locating a brain activity, implemented by a method according to one of the preceding claims; and
   - a step of determining optimum locations of said brain activity sensors according to the results of said step of locating a brain activity.

FIG. 1

FIG. 2A

FIG. 2B

FIG. 2C

FIG. 3

FIG. 4A

FIG. 4B

FIG. 5A

FIG. 5B

FIG. 5C

FIG. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **J. MELLINGER et al.** An MEG-based Brain-Computer Interface (BCI). *Neuroimage,* 01 Juillet 2007, vol. 36 (3), 581-593
- **A. BARACHANT ; T. AKSENOVA ; S. BONNET.** Filtrage spatial robuste à partir d'un sous-ensemble optimal d'électrodes en BCI EEG. *GRETSI 2009,* Septembre 2009, 8-11
- **F. TADEL et al.** Brainstorm : A User-Friendly Application for MEG/EEG Analysis. *Computational Intelligence and Neuroscience,* 2011, vol. 2011
- **BOURGUIGNON et al.** Neuronal network coherent with hand kinematics during fast repetitive hand movements. *NeuroImage,* 2012, vol. 59, 1684-1691
- **J. VRBA ; S. E. ROBINSON.** Signal Processing in Magnetoencephalography. *Methods,* 2001, vol. 25, 249-271